# EUROPEAN PATENT APPLICATION

(11) **EP 4 098 199 A1**
(43) Date of publication of application: **07.12.2022**
(21) Application number: 21177112.6
(22) Date of filing: 01.06.2021
(51) Int. Cl.: A61B 6/00, G16H 30/40, G16H 40/60, G06T 7/00, G16H 30/20, G16H 50/20

(54) **APPARATUS FOR MEDICAL IMAGE ANALYSIS**

(71) Applicant: Koninklijke Philips N.V., 5656 AG Eindhoven (NL)
(72) Inventor: GRASS, Michael, 5656 AE Eindhoven (NL); BALTRUSCHAT, Ivo Matteo, 5656 AE Eindhoven (NL); SAALBACH, Axel, 5656 AE Eindhoven (NL)
(74) Representative: Philips Intellectual Property & Standards

(57) **Abstract**

The present invention relates to an apparatus (10) for medical image analysis, comprising: a camera (20); a processing unit (30); and an output unit (40). The camera is configured to be placed in proximity of a system image display of a medical imaging system. The camera is configured to acquire a local image of a system image displayed on the system image display, wherein the system image comprises medical image data of a patient and wherein the local image comprises local image data of the medical image data of the patient. The processing unit is configured to determine a plurality of image and imaging parameters, the determination comprising utilization of the local image. The processing unit is configured utilize the plurality of image and imaging parameters to determine a process decision to:
either
determine if the local image data of the medical image data is suitable for further processing;
or
determine if a new local image is to be acquired and a new plurality of image and imaging parameters determined for the new local image and the new plurality of image and imaging parameters utilized to determine a new process decision.
The output unit is configured to output image data.

## Description

### FIELD OF THE INVENTION

The present invention relates to an apparatus for medical image analysis, a method for medical image analysis, as well as to a computer program element and a computer readable medium.

### BACKGROUND OF THE INVENTION

In recent years, AI-based image analysis showed promising results in a broad range of applications from computer vision tasks to medical image analysis.

However, medical devices such as X-Ray systems or diagnostic applications are often used in a secure environment (e.g. without internet access) and as such the AI-based image analysis software can become out of date because it cannot be frequently updated due to quality and regulatory requirements.

There is a need to resolve this issue.

### SUMMARY OF THE INVENTION

It would be advantageous to have an improved means of analyzing medical images. The object of the present invention is solved with the subject matter of the independent claims, wherein further embodiments are incorporated in the dependent claims. It should be noted that the following described aspects and examples of the invention apply to the apparatus for medical image analysis, and also to the method for medical image analysis as well as to the computer program element and the computer readable medium.

In a first aspect, there is provided an apparatus for medical image analysis, comprising:
- a camera;
- a processing unit; and
- an output unit.

The camera is configured to be placed in proximity of a system image display of a medical imaging system. The camera is configured to acquire a local image of a system image displayed on the system image display, where the system image comprises medical image data of a patient and the local image comprises local image data of the medical image data of the patient. The processing unit is configured to determine a plurality of image and imaging parameters, the determination comprising utilization of the local image. The processing unit is configured utilize the plurality of image and imaging parameters to determine a process decision to:
either
determine if the local image data of the medical image data is suitable for further processing;
   or
determine if a new local image is to be acquired and a new plurality of image and imaging parameters determined for the new local image and the new plurality of image and imaging parameters utilized to determine a new process decision;
   and

The output unit is configured to output image data.

In an example, if the local image data of the medical image data is determined to be suitable for further processing, the processing unit is configured to implement a machine learning algorithm to generate local processed image data, wherein the generation comprises utilization of the local image data of the medical image data of the patient. The output unit is configured to output the local processed image data

Thus, the diagnosis of conditions a patient may have captured in medical image from medical imaging device or system, such as an X-ray system or MRI is supported using screen captures of the displays of the medical imaging device or system.

Prior to implementation of a machine learning algorithm, such as a trained neural network, certain imaging parameters such as distance between a smart phone and a display of the medical imaging device, and indeed display settings on the display of medical imaging device, and image quality are utilised to determine if the machine learning algorithm can operate upon image acquired by the smart phone, or if the smart phone should be repositioned to acquire new image and/or display of the medical imaging device be adjusted in order that the smart phone acquires a more appropriate image to then be analysed by the machine learning algorithm operating upon the smart phone.

In other words, acquisition conditions of a smart phone camera acquisition are detected of an image of a medical image display prior to the application of a neural network for disease detection where the expected image quality of the smart phone camera image is estimated and advice can be given to a user to change the acquisitions settings and to retake the image where confidence level or certainty indicator information that are related to the detected diseases according to image quality can be displayed, or the neural network analysis can operate on the smart phone image, where the acquisition conditions can be integrated into the neural network analysis. In this manner, image quality is improved and AI disease detection is achieved.

It is to be noted that the system image display of the medical imaging system need not be attached to a medical imaging unit, but could be separated from the medical imaging unit and could indeed be part of a PACS display. Thus, the system image display does not need to be showing a live image of the patient, but can be showing a saved image of the image. However, the medical image display can be showing a current image of a patient that has relatively recently been acquired. This explains what is meant by "medical imaging system" and the "system image display".

In this manner, diagnostic accuracy can be improved and specific machine learning algorithms can be utilised.

In an example, generation of the local processed image data comprises utilization of the plurality of image and imaging parameters to calculate error information.

In an example, the processing unit is configured to utilize the plurality of image and imaging parameters to calculate at least one certainty indicator or confidence level for the local processed image data.

In an example, the processing unit is configured to generate a certainty indicator or confidence level heat map superimposed upon the location processed image data.

Thus, imaging parameters such as the quality of the image acquired by the smart phone or other imaging apparatus, can be taken into account enabling the user looking at the processed image to understand if parts of the image have a higher error associated with any determined diagnosis. Thus, for example if there was glare on the part of the medical imaging system display, that was picked up by the smart phone, this part of the processed image could have a higher error associated with it than other parts of the image enabling a user to better interpret the processed image. The same applies to signal-to-noise across the image, and parameters such as distance from the camera to different parts of the medical image system display.

In an example, implementation of the machine learning algorithm comprises utilization of the plurality of image and imaging parameters.

In an example, the processing unit is configured to adapt the machine learning algorithm, wherein the adaptation comprises utilization of the plurality of image and imaging parameters.

Thus, the specific machine learning algorithm, such as a neural network, can be selected based upon the image and imaging parameters enabling the best machine learning algorithm or most appropriate machine learning algorithm to be utilised. Furthermore, a selected or a default machine learning algorithm can be adapted based on the image and imaging parameters in order that it is optimised for analysis of the image data acquired on the smart phone or other equivalent apparatus.

In an example, the processing unit is configured to utilize the plurality of image and imaging parameters to determine a new distance between the camera and the system image display for acquisition of the new local image, and wherein the output unit is configured to output information relating to the new distance.

In an example, the processing unit is configured to utilize the plurality of image and imaging parameters to determine a new angle between a viewing axis of the camera and an axis perpendicular to the system image display for acquisition of the new local image, and wherein the output unit is configured to output information relating to the new angle.

In this manner, the apparatus such a smart phone can use knowledge of the display screen size of the medical imaging system to determine the distance to the display screen, for example determined from the outer extent of the display screen. This can also be used to determine the angle between the camera and the display screen. The smart phone or other apparatus can have more than one camera, and triangulation used to determine distance to the display screen or other standard distance determining methodologies utilised by smart phones. The processing unit can then determine that these parameters are not optimum with respect to the acquired image by the smart phone, and present to the user information such as the position the smart phone closer to the medical system display and/or a different angle such as more face on the display in order to enable a better image to be acquired by the smart phone.

In an example, the processing unit is configured to utilize the plurality of image and imaging parameters to determine at least one new display setting for the system image display for acquisition of the new local image, and the output unit is configured to output information relating to the new display setting.

Thus, the processing unit can implement image processing software with for example text and icon identification capabilities, enabling display settings such as resolution, contrast, brightness, of the medical system display to be determined. The smart phone or other similar apparatus can then provide information to the user to adjust the display settings on the medical system display in order that a better and more appropriate image can be acquired by the smart phone of the image data being presented on the medical image display.

In an example, the processing unit is configured to utilize the plurality of image and imaging parameters to determine a change in lighting conditions in a room within which the system image display is located, and the output unit is configured to output information relating to the change in lighting conditions.

From the smart phone image of the medical image display, or other apparatus image of the medical image display, image processing enables it to be determined if there is glare for example on the display screen caused by lighting or sunlight coming through a window for example. The determination can then be made that this is leading to non-optimum image data acquired by the smart phone, and information can be relayed to the user to adjust the lighting conditions in the room within which the medical image display is situated.

In an example, the plurality of image and imaging parameters comprises two or more of: determined distance between the camera and the system image display; determined angle between a viewing axis of the camera and an axis perpendicular to the system image display; determined signal to noise at one or more locations in the local image; determined one or more lighting conditions in a room within which the system image display is located; determined display setting for the system image display.

In a second aspect, there is provided a method for medical image analysis, comprising:
a) placing a camera of an apparatus in proximity of a system image display of a medical imaging system;
b) acquiring by the camera a local image of a system image displayed on the system image display, wherein the system image comprises medical image data of a patient and wherein the local image comprises local image data of the medical image data of the patient;
c) determining by a processing unit of the apparatus a plurality of image and imaging parameters, the determining comprising utilizing the local image;
d) determining a process decision by the processing unit, the determining comprising utilizing the plurality of image and imaging parameters, and wherein the process decision is to:
   either
   determine if the local image data of the medical image data is suitable for further processing;
      or
   determine if a new local image is to be acquired and a new plurality of image and imaging parameters determined for the new local image and the new plurality of image and imaging parameters utilized to determine a new process decision;
      and
f) outputting by an output unit of the apparatus image data.

In an example, if the local image data of the medical image data is determined to be suitable for further processing the method comprises:
e) generating local processed image data by implementing by the processing unit a machine learning algorithm to generate the local processed image data, wherein the generating comprises utilizing the local image data of the medical image data of the patient; and
wherein step f) comprises outputting by the output unit the local processed image data.

According to another aspect, there is provided a computer program element controlling one or more of the apparatuses as previously described which, if the computer program element is executed by a processing unit, is adapted to perform the method as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic set up of an example of an apparatus for medical image analysis;
Fig. 2 shows a method for medical image analysis; and
Fig. 3 shows a smart phone acquiring an image of a medical image shown on a display of a medical imaging system/device.

### DETAILED DESCRIPTION OF EMBODIMENTS

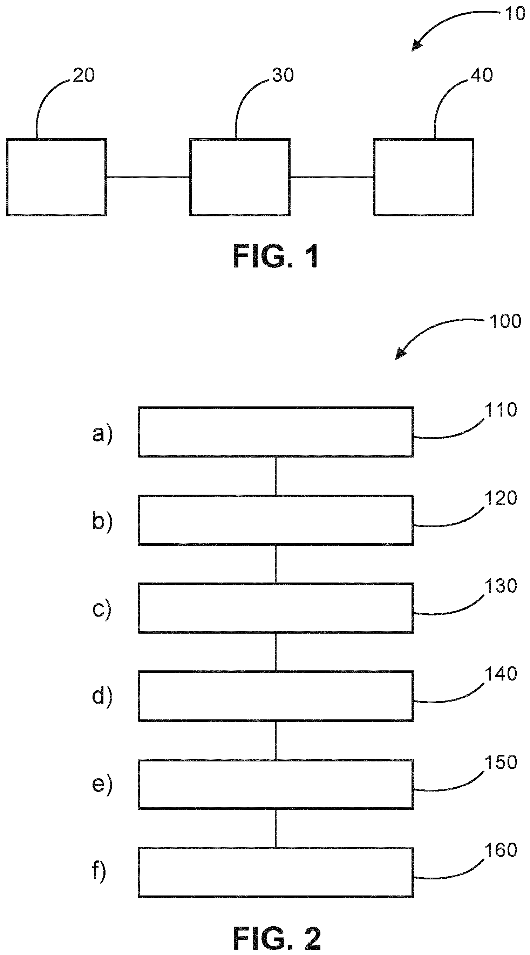

Fig. 1 shows a schematic example of an apparatus 10 for medical image analysis. The apparatus comprises a camera 20, a processing unit 30, and an output unit 40. The camera is configured to be placed in proximity of a system image display of a medical imaging system. The camera is configured to acquire a local image of a system image displayed on the system image display. The system image comprises medical image data of a patient and the local image comprises local image data of the medical image data of the patient. The processing unit is configured to determine a plurality of image and imaging parameters, the determination comprising utilization of the local image. The processing unit is configured utilize the plurality of image and imaging parameters to determine a process decision to:
either
determine if the local image data of the medical image data is suitable for further processing;
   or
determine if a new local image is to be acquired and a new plurality of image and imaging parameters determined for the new local image and the new plurality of image and imaging parameters utilized to determine a new process decision.

The output unit is configured to then output image data.

According to an example, if the local image data of the medical image data is determined to be suitable for further processing, the processing unit is configured to implement a machine learning algorithm to generate local processed image data. The generation of the local processed image data can then comprise utilization of the local image data of the medical image data of the patient. The output image data output by the output unit can then comprise the local processed image data.

In an example, the local processed image data is an indication of a disease classification.

In an example, the local processed image data comprises an indication of a disease classification.

In an example, the local processed image data comprises a diagnosis.

Thus, for example an indication of a lung nodule being present or an indication of cancer or an indication of another medical condition or an indication of a broken bone or other condition that can be indicated on the basis of medical imaging can be presented. This can be in the form of a simple textual yes/no, that can have an associated confidence level or certainty indicator. This can also be in the form of an image corresponding to the local image with the disease/medical condition classification superimposed upon the image, indicating the location of the determined classification.

In an example, the apparatus is a smart phone.

In an example, the processing unit can be at a separate location to the computer. For example, the camera can acquire imagery that is transmitted to a processing unit over a network, and where that processing unit could be within the cloud. The processed information can then be sent back to the operator of the camera. This means that the decision to acquire a new image or proceed with the image just acquired can be made locally, or be made remotely. Also, the processing of the image data itself to provide a medical analysis can again be carried out remotely or locally, and the information provided back to the operator if necessary.

In an example, the apparatus is configured to download over a network an update to the machine learning algorithm, and update the machine learning algorithm based on the update.

In an example, the apparatus is configured to download over a network a new machine learning algorithm, and replace the old machine learning algorithm with the new machine learning algorithm and the machine learning algorithm utilized by the apparatus becomes the new machine learning algorithm.

In an example, the output unit comprises a display screen of the apparatus.

In an example, generation of the local processed image data comprises utilization of the plurality of image and imaging parameters.

In an example, plurality of image and imaging parameters comprises a display parameter relating to the system image on the system image display, such as a contrast or grey scale level or dynamic range.

In an example, the processing unit is configured to implement an image processing algorithm to determine the display parameter.

Thus, the processing unit can determine from a markers associated with the image, such as a written grey scale value or a grey scale indicate in a bar form, an image and imaging parameters associated with how the image has been displayed. In this way, if it is determined that the local image is not good enough for further processing or could be improved, information could be provided to an operator to increase the dynamic range and/or contrast of what is being displayed on the system display and then re-taking an image with the camera.

In an example, the processing unit is configured to implement a neural network algorithm to determine the display parameter.

Thus, a series of images of different contrast levels and dynamic range levels can be acquired and used to train a neural network, where ground truth information is provided regarding the contrast level / dynamic range for the training images. Then, when an operator acquires an image of what is being presented on the display of the medical image unit, on the basis of the content within the image itself a determination can be made as to whether the image could be improved by adjusting the output setting of the medical image display unit.

According to an example, the processing unit is configured to utilize the plurality of image and imaging parameters to calculate at least one certainty indicator or confidence level for the local processed image data.

According to an example, the processing unit is configured to utilize the plurality of image and imaging parameters to calculate a plurality of certainty indicators or confidence levels for a plurality of locations of the local processed image data.

Thus, confidence levels regarding disease classification can be provided, which provides the operator with a better interpretation and understanding of the analysis of the image. It would also be understood by the skilled person that a "certainty indicator" can also be construed to be an "uncertainty indicator", in that a certainty indicator of a diagnosis being correct, implicitly provides an uncertainty indicator relating to whether the diagnosis is correct.

According to an example, the processing unit is configured to generate a certainty indicator or confidence level heat map superimposed upon the location processed image data.

According to an example, implementation of the machine learning algorithm comprises utilization of the plurality of image and imaging parameters.

In an example, implementation of the machine learning algorithm comprises selecting the machine learning algorithm from a plurality of machine learning algorithms, the selection comprising utilization of the plurality of image and imaging parameters.

According to an example, the processing unit is configured to adapt the machine learning algorithm, wherein the adaptation comprises utilization of the plurality of image and imaging parameters.

According to an example, the processing unit is configured to utilize the plurality of image and imaging parameters to determine a new distance between the camera and the system image display for acquisition of the new local image. The output unit is configured to output information relating to the new distance.

According to an example, the processing unit is configured to utilize the plurality of image and imaging parameters to determine a new angle between a viewing axis of the camera and an axis perpendicular to the system image display for acquisition of the new local image. The output unit is configured to output information relating to the new angle.

According to an example, the processing unit is configured to utilize the plurality of image and imaging parameters to determine at least one new display setting for the system image display for acquisition of the new local image. The output unit is configured to output information relating to the new display setting.

According to an example, the processing unit is configured to utilize the plurality of image and imaging parameters to determine a change in lighting conditions in a room within which the system image display is located. The output unit is configured to output information relating to the change in lighting conditions.

According to an example, the plurality of image and imaging parameters comprises two or more of: determined distance between the camera and the system image display; determined angle between a viewing axis of the camera and an axis perpendicular to the system image display; determined signal to noise at one or more locations in the local image; determined one or more lighting conditions in a room within which the system image display is located; determined display setting for the system image display.

In an example, the machine learning algorithm is a trained neural network.

In an example, the neural network is trained using a plurality of medical images of patients and associated ground truth information relating to diagnosis of conditions, diseases, made by medical experts.

In an example, the neural network is trained using image and imaging parameters associated with images used for training. Thus, the neural network can be trained based on images acquired by a camera of a medical image presented on a screen along with the image and imaging parameters. Then the neural network can operate more effectively, when analysing a new image. The generation of the local processed data for a new image can then make use of the local image data of the medical image data of the patient and the plurality of image and imaging parameters associated with the acquisition of the local image.

Also, the skilled person would understand that an overall system can be formed or provided, where the overall system comprises the apparatus as described above with respect to Fig. 1 and also comprises the medical imaging system.

Fig. 2 shows a method 100 for medical image analysis, where step e) is optional. The method comprises:
in a placing step 110, also referred to as step a), placing a camera of an apparatus in proximity of a system image display of a medical imaging system;
in an acquiring step 120, also referred to as step b), acquiring by the camera a local image of a system image displayed on the system image display, wherein the system image comprises medical image data of a patient and wherein the local image comprises local image data of the medical image data of the patient;
in a determining step 130, also referred to as step c), determining by a processing unit of the apparatus a plurality of image and imaging parameters, the determining comprising utilizing the local image;
in a determining step 140, also referred to as step d), determining a process decision by the processing unit, the determining comprising utilizing the plurality of image and imaging parameters, and wherein the process decision is to:
   either
   determine if the local image data of the medical image data is suitable for further processing;
      or
   determine if a new local image is to be acquired and a new plurality of image and imaging parameters determined for the new local image and the new plurality of image and imaging parameters utilized to determine a new process decision;
      and
   in an outputting step 160, also referred to as step f), outputting by an output unit of the apparatus image data.

According to an example, wherein if the local image data of the medical image data is determined to be suitable for further processing the method comprises:
in a generating step 150, also referred to as step e), generating local processed image data by implementing by the processing unit a machine learning algorithm to generate the local processed image data, wherein the generating comprises utilizing the local image data of the medical image data of the patient; and
wherein step f) comprises outputting by the output unit the local processed image data.

In an example, the apparatus is a smart phone.

In an example, the method comprises downloading to the apparatus over a network an update to the machine learning algorithm, and updating the machine learning algorithm based on the update.

In an example, the method comprises downloading to the apparatus over a network a new machine learning algorithm, and replacing the old machine learning algorithm with the new machine learning algorithm and the machine learning algorithm utilized by the apparatus becomes the new machine learning algorithm.

In an example, the output unit comprises a display screen of the apparatus.

In an example, generating of the local processed image data comprises utilizing the plurality of image and imaging parameters to calculate error information.

In an example, the method comprises utilizing by the processing unit the plurality of image and imaging parameters to calculate at least one confidence level or certainty indicator for the local processed image data.

In an example, the method comprises utilizing by the processing unit the plurality of image and imaging parameters to calculate a plurality of confidence levels or certainty indicators for a plurality of locations of the local processed image data.

In an example, the method comprises generating by the processing unit a confidence level or certainty indicator heat map superimposed upon the location processed image data.

In an example, implementing the machine learning algorithm comprises utilizing the plurality of image and imaging parameters.

In an example, implementing the machine learning algorithm comprises selecting the machine learning algorithm from a plurality of machine learning algorithms, the selecting comprising utilizing the plurality of image and imaging parameters.

In an example, the method comprises adapting by the processing unit the machine learning algorithm, wherein the adapting comprises utilizing the plurality of image and imaging parameters.

In an example, the method comprises utilizing by the processing unit the plurality of image and imaging parameters to determine a new distance between the camera and the system image display for acquisition of the new local image, and outputting by the output unit information relating to the new distance.

In an example, the method comprises utilizing by the processing unit the plurality of image and imaging parameters to determine a new angle between a viewing axis of the camera and an axis perpendicular to the system image display for acquisition of the new local image, and outputting by the output unit information relating to the new angle.

In an example, the method comprises utilizing by the processing unit the plurality of image and imaging parameters to determine at least one new display setting for the system image display for acquisition of the new local image, and outputting by the output unit information relating to the new display setting.

In an example, the method comprises utilizing by the processing unit the plurality of image and imaging parameters to determine a change in lighting conditions in a room within which the system image display is located, and outputting by the output unit information relating to the change in lighting conditions.

In an example, the plurality of image and imaging parameters comprises two or more of: determined distance between the camera and the system image display; determined angle between a viewing axis of the camera and an axis perpendicular to the system image display; determined signal to noise at one or more locations in the local image; determined one or more lighting conditions in a room within which the system image display is located; determined display setting for the system image display.

In an example, the machine learning algorithm is a trained neural network.

Therefore, a medical imaging unit/system is operating in a normal way, and obtaining a medical image, also called a system image, that is displayed on a display of the medical imaging unit/system. However, the image analysis software provided with the medical imaging unit/system may be out of date and not exhibit functionalities available with the most up-to-date software. To mitigate this a smart phone acquires an image of what is being displayed on the unit/system display, and this image is analysed by an up-to date AI algorithm to perform a gold standard diagnosis. However, prior to the AI operating on the image, a determination is made if the smart phone acquired image is suitable for such AI processing, and if not feedback is given to the user in terms of how to obtain a better, suitable, image of what is being displayed on the unit/system display. This is facilitated through the constantly improving smart phone hardware (i.e. camera resolution, computing power), and the increasing availability of highspeed internet connections, enabling it to be possible to employ (always up-to-date) AI applications in the daily routine. It is however to be noted, that the smart phone can just acquire the image and transmit this to another location, such as the cloud, where the processing is undertaken. Thus here the "apparatus" can be a single unit of camera, processing unit and output unit or be formed from separate units, that can be in completely separate locations. However, for simplicity the following discussion centres on the apparatus in the form of a smart phone, but as detailed above the discussion is not limited to that specific embodiment, which simply forms an example of one form of the apparatus.

Therefore, the use of smart phone provides an interesting alternative to bring AI technology into the clinical environment (especially in developing countries), where measured viewing parameters of the smart phone camera images and information derived regarding the unit/system display can be integrated in the AI based image classification.

It is to be noted that a camera can acquire the image of what is being presented on the unit/system display and this can be transmitted over a network to a processor that carries out the determination that the image is ok for further processing or that a new image needs to be acquired, and also carry out the further processing. Such, a processor can be in the cloud for example.

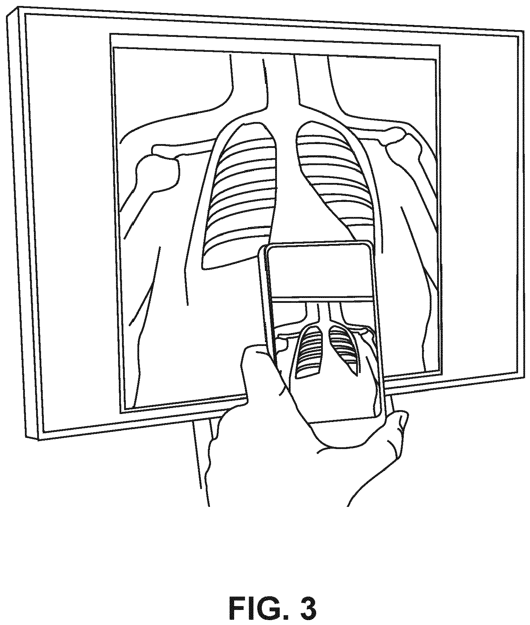

The apparatus for medical image analysis and method for medical image analysis are explained in specific detail, where a smart phone is described, but as made clear above, this is only exemplary, and where reference is made to Fig. 3.

Fig. 3 shows an example of a smart phone acquiring a local image of an image being displayed on a display or monitor of a medical image acquisition unit or system, which in this case is an X-ray unit but could be an MRI unit or PET unit or any other medical imaging unit. The smart phone then processes the image to give feedback, if necessary, regarding how to take a new, better image, which could be re-positioning of the smart phone, changing setting on the monitor, and/or changing lighting conditions in the room. The newly acquired image, or the original image if it was already suitable for further processing, is then provided to a trained neural network operating on the smart phone, where that neural network can be frequently updated through downloaded updates. The neural network can also utilize the acquired acquisition parameters to operate more effectively, and indeed a specific neural network from several stored on the smart phone can be chosen based on the acquisition parameters.

Continuing with Fig. 3, a detailed workflow is now discussed:
As shown in Fig. 3 the smart phone is positioned relative to a medical unit display/monitor and has acquired a local image of the system image displayed. A number of different image acquisition parameters are detected regarding the smartphone image acquisition, that will be utilized in disease detection. These include but are not limited to:
The distance and acquisition angle of the smart phone - strong angulations of the smart phone relative to the medical image display lead to image distortions while a large distance results in an information loss.

This can be measured for example via the display frame, text on the display etc.

The overall image quality. This can be determined from the quality of the display and the smart phone camera.

This can be measured by overall image quality parameters like sharpness, noise, signal to noise ratio (SNR), contrast to noise ratio (CNR) etc.

The level and window of the displayed images

This can be estimated via typical grey value distributions in the images or derived from the text on the display. This can be determined by image processing software obtaining this information from monitor settings displayed on the unit/system display for example.

Room lighting and reflection spots on the display

This can be determined from the overall brightness of the image or bright spots in the image

It is to be noted that the overall image quality and the setting for the unit/system display, in terms of an indication of the dynamic range/contrast can be determined by a trained neural network. Thus, many images with ground truth information of monitor setting, such as grey scale settings and/or contrast levels can be used to train a NN When an image of the unit/system is acquired, a determination can be made of the display modality for the display of the unit/system. If a determination is made that the acquired image is not commensurate with further processing, one form of feedback can then be, if appropriate, for an operator to increase the dynamic range / contrast settings for the unit/system display for example.

The determined parameters are then used for three different purposes:
Advice is given to the user of the smart phone if and how to optimize the image acquisition with the smart phone and how to minimize the loss in image quality (for example by showing a positioning instructions in the interface / the current image quality level)

When displaying the results of the neural network for disease detection on the smart phone the achieved results can be modified with a corresponding accuracy value / uncertainty. In the situation where regional or local image quality degradation is taking place, the heat map of the neural network can be correlated to the image quality map and either both are visualized on a per disease basis or disease certainty numbers are calculated.

Finally, image quality adapted neural networks can be applied that can take account of the measured image acquisition settings / quality parameters or the parameters can be integrated into the training and the inference of the network.

Thus, it could be expected that the use of smart phones to acquire imagery of medical image displays for applications such as chest X-ray analysis could be problematic due to the loss of image quality that would be expected to be an inherent limitation in such scenarios, combined with the display of diagnostic X-ray images on the monitor or display of the medical image unit itself being non-optimum resulting in a loss information, for example due to the selection of specific windows level settings, resizing, contrast, brightness etc. Also, it might be expected that environment conditions such as lighting, positioning of the smart phone, and the characteristics of the camera could affect the image which would be subject to an AI-based analysis, leading to an expected substantial deterioration of the image quality, and AI performance. However, the present system and method addresses this through detection of the acquisition conditions of the smart phone camera prior to the application of the neural network for disease detection. The expected image quality of the camera image is estimated and either advice is given to the user to change the acquisitions settings and to retake the image, and where AI analysis is undertaken for example error bars are presented related to the detected diseases are presented according to image quality at different image locations, and the acquisition conditions can also be integrated into the AI neural network analysis to provide a more optimized AI analysis.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus (10) for medical image analysis, comprising:
- a camera (20);
- a processing unit (30); and
- an output unit (40);
wherein the camera is configured to be placed in proximity of a system image display of a medical imaging system;
wherein the camera is configured to acquire a local image of a system image displayed on the system image display, wherein the system image comprises medical image data of a patient and wherein the local image comprises local image data of the medical image data of the patient;
wherein the processing unit is configured to determine a plurality of image and imaging parameters, the determination comprising utilization of the local image;
wherein the processing unit is configured utilize the plurality of image and imaging parameters to determine a process decision to:
either
determine if the local image data of the medical image data is suitable for further processing;
or
determine if a new local image is to be acquired and a new plurality of image and imaging parameters determined for the new local image and the new plurality of image and imaging parameters utilized to determine a new process decision;
and
wherein the output unit is configured to output image data.

2. Apparatus according to claim 1, wherein if the local image data of the medical image data is determined to be suitable for further processing, the processing unit is configured to implement a machine learning algorithm to generate local processed image data, wherein the generation comprises utilization of the local image data of the medical image data of the patient; and
wherein the output unit is configured to output the local processed image data.

3. Apparatus according to claim 2, wherein generation of the local processed image data comprises utilization of the plurality of image and imaging parameters to calculate error information.

4. Apparatus according to claim 3, wherein the processing unit is configured to utilize the plurality of image and imaging parameters to calculate at least one certainty indicator or confidence level for the local processed image data.

5. Apparatus according to any of claims 3-4, wherein the processing unit is configured to generate an certainty indicator or confidence level heat map superimposed upon the location processed image data.

6. Apparatus according to any of claims 2-5, wherein implementation of the machine learning algorithm comprises utilization of the plurality of image and imaging parameters.

7. Apparatus according to claim 6, wherein the processing unit is configured to adapt the machine learning algorithm, wherein the adaptation comprises utilization of the plurality of image and imaging parameters.

8. Apparatus according to any of claims 1-7, wherein the processing unit is configured to utilize the plurality of image and imaging parameters to determine a new distance between the camera and the system image display for acquisition of the new local image, and wherein the output unit is configured to output information relating to the new distance.

9. Apparatus according to any of claims 1-8, wherein the processing unit is configured to utilize the plurality of image and imaging parameters to determine a new angle between a viewing axis of the camera and an axis perpendicular to the system image display for acquisition of the new local image, and wherein the output unit is configured to output information relating to the new angle.

10. Apparatus according to any of claims 1-9, wherein the processing unit is configured to utilize the plurality of image and imaging parameters to determine at least one new display setting for the system image display for acquisition of the new local image, and wherein the output unit is configured to output information relating to the new display setting.

11. Apparatus according to any of claims 1-10, wherein the processing unit is configured to utilize the plurality of image and imaging parameters to determine a change in lighting conditions in a room within which the system image display is located, and wherein the output unit is configured to output information relating to the change in lighting conditions.

12. Apparatus according to any of claims 1-11, wherein the plurality of image and imaging parameters comprises two or more of: determined distance between the camera and the system image display; determined angle between a viewing axis of the camera and an axis perpendicular to the system image display; determined signal to noise at one or more locations in the local image; determined one or more lighting conditions in a room within which the system image display is located; determined display setting for the system image display.

13. A method (100) for medical image analysis, comprising:
a) placing (110) a camera of an apparatus in proximity of a system image display of a medical imaging system;
b) acquiring (120) by the camera a local image of a system image displayed on the system image display, wherein the system image comprises medical image data of a patient and wherein the local image comprises local image data of the medical image data of the patient;
c) determining (130) by a processing unit of the apparatus a plurality of image and imaging parameters, the determining comprising utilizing the local image;
d) determining (140) a process decision by the processing unit, the determining comprising utilizing the plurality of image and imaging parameters, and wherein the process decision is to:
either
determine if the local image data of the medical image data is suitable for further processing;
or
determine if a new local image is to be acquired and a new plurality of image and imaging parameters determined for the new local image and the new plurality of image and imaging parameters utilized to determine a new process decision;
and
f) outputting (160) by an output unit of the apparatus image data.

14. Method according to claim 13, wherein if the local image data of the medical image data is determined to be suitable for further processing the method comprises:
e) generating (150) local processed image data by implementing by the processing unit a machine learning algorithm to generate the local processed image data, wherein the generating comprises utilizing the local image data of the medical image data of the patient; and
wherein step f) comprises outputting (160) by the output unit the local processed image data.

15. A computer program element for controlling a system according to any one of claims 1 to 13, which when executed by a processor is configured to carry out the method of claim 14.
